# EUROPEAN PATENT SPECIFICATION

(11) **EP 1 991 503 B1**
(45) Date of publication and mention of the grant of the patent: **08.09.2010**
(21) Application number: 07711106.0
(22) Date of filing: 23.02.2007
(51) Int. Cl.: C01G 49/08, C01G 49/06, A61K 49/18, C09C 1/24

(54) **METHOD OF PREPARATION OF SUPERPARAMAGNETIC NANOPARTICLES BASED ON IRON OXIDES WITH MODIFIED SURFACE AND SUPERPARAMAGNETIC NANOPARTICLES OBTAINED BY SUCH A METHOD**
VERFAHREN ZUR HERSTELLUNG VON SUPERPARAMAGNETISCHEN NANOPARTIKELN AUF BASIS VON EISENOXIDEN MIT MODIFIZIERTER OBERFLÄCHE UND NACH DIESEM VERFAHREN HERGESTELLTE SUPERPARAMAGNETISCHE NANOPARTIKEL
PROCÉDÉ DE PRÉPARATION DE NANOPARTICULES SUPERPARAMAGNÉTIQUES À BASE D'OXYDES DE FER AYANT UNE SURFACE MODIFIÉE ET NANOPARTICULES SUPERPARAMAGNÉTIQUES OBTENUES PAR LEDIT PROCÉDÉ

(30) Priority: 24.02.2006 CZ 20060120
(43) Date of publication of application: 19.11.2008
(73) Proprietor: Ustav Makromolekularni Chemie Akademie Ved Ceske Republiky, v.v.i, 16206 Praha 6 (CZ); Ustav Experimentalni Mediciny Akademie Ved Ceske Republiky, v.v.i., 142 20 Praha 4 (CZ)
(72) Inventor: HORAK, Daniel, 152 00 Praha 5 (CZ); SYKOVA, Eva, 162 00 Praha 6 (CZ); BABIC, Michal, 278 32 Zborovice,okr.Kromeriz (CZ); JENDELOVA, Pavla, 252 68 Stredokluky (CZ); HAJEK, Milan, 148 00 Praha 4 (CZ)
(74) Representative: Beetz & Partner
(86) International application number: PCT/CZ2007/000012
(87) International publication number: WO 2007/095871

(56) References cited:
- EP-A2- 0 516 252
- WO-A-91/02811
- WO-A-97/35200
- DE-A1- 2 642 383
- US-A- 4 101 435
- US-A- 4 452 773
- US-A- 4 827 945
- US-A- 5 492 814
- US-A1- 2005 271 593

## Description

### Technical field

The invention concerns a method of preparation of superparamagnetic nanoparticle probes based on iron oxides with modified surface and superparamagnetic nanoparticles obtained by such a method.

### Background art

The development of medical diagnostics in recent years aims more and more at earlier diagnosis of frequently very serious diseases. A part of the new techniques is cell labelling or cell imaging by magnetic resonance. Magnetic resonance imaging (MRI) makes it possible to visualize internal organs of humans and hence is a great contribution not only in diagnostics but also in therapy and surgery. Medical diagnostics requires the use of nanometre particles. MRI makes use of the fact that magnetic nanoparticles create a magnetic field and influence the environment (Shinkai M., Functional magnetic particles for medical application, J. Biosci Bioeng 94, 606-613, 2002). The range of particle sizes can be divided, depending on application, into "large" (diameter > 50 nm) and "small" (diameter < 50 nm) particles. MR diagnostics of liver and spleen is their main application field as the particles of this size are readily and almost completely taken up by the macrophages of these organs (Kresse M., Pfefferer D., Lawaczeck R., EP 516,252 A2; Groman E.V., Josephson L., U.S. Pat. 4,770,183). The particles find applications also in clinical hyperthermia (Hasegawa M., Nagae H., Ito Y., Mizutani A., Hirose K., Ohgai M., Yamashita Y., Tozawa N., Yamada K, Kito K, Hokukoku S., WO 92/22586 A1; Gordon R.T., U.S. Pat. 4,731,239).

For labelling of cells it is of key importance to prepare monocrystalline nanoparticles of an iron oxide dispersible in water, which are also biocompatible, superparamagnetic, surface functionalizable and which are, at the same time, completely taken up by the cells.

At present, superparamagnetic iron oxides (without magnetic memory) are the class of materials with the strongest contrast in MR (Gromann E.V., Josephson L., Lewis J. M., US 4 827 945 A; Stark D.D., Weissleder R., Elizondo G., Hahn P.F., Saini S., Todd L.E., Wittenberg J., Ferrucci J.T., Superparamagnetic iron oxide: clinical application as a contrast agent for MR imaging of the liver, Radiology 168, 297-301, 1988), hence they are in low concentrations especially suitable for tissue-specific applications. A critical size namely exists, below which the particles can have only a single magnetic domain even in zero magnetic field. The condition for superparamagnetism is *KV* ∼ *kT,* where *KV* is the anisotropy energy (*K* is the anisotropy constant, V is the particle volume) and *k T* is the thermal energy of motion (k is the Boltzmann constant, T is absolute temperature). If this condition is fulfilled, particle magnetization can be caused by thermal energy *kT* provided that it exceeds the potential barrier of anisotropic energy. The critical size of superparamagnetic particles of magnetite is ca. 25 nm. Superparamagnetic iron oxides make it possible to enhance the tissue contrast by increasing the relaxation rates of water. Varying the size, coating, thickness, surface chemical reactions and targeting ligands, the nanoparticle probes can be targeted on specific organs and cells or can even become *in vivo* molecular markers for various diseases. However, the size of crystal core of iron oxides, which causes a specific character to the materials, is problematic because it shows an essential influence on biological behavior. A small size of the particles improves their precise targeting but the efficiency of the material decreases due to interdependence of the particle size and magnetic moment. As a consequence, it is necessary to seek a compromise between good contrast effect of the material and precise targetability (Kresse M., Pfefferer D., Lawaczeck R., Wagner S. Ebert W., Elste V., Semmler W., Taupitz M. Gaida J., Herrmann A., Ebert M., Swiderski U., U.S. Pat. Appl. 2003,0185757). As a rule, the iron-containing core should be as large as possible to obtain a high imaging effect (contrast), but the overall diameter should be small.

Examples of MRI contrast agents include injectable nuclei, radionuclides, diamagnetic, paramagnetic, ferromagnetic, superparamagnetic materials, contrast materials containing iron (e:g., iron oxide, iron(III) ions, ammonium iron(III) citrate), gadolinnium agents (e.g. gadolinium diethylenetriaminepentaacetate) and manganese paramagnetic materials. Typical commercial MRI contrast agents are, e.g., Magnevist^{®} and Resovist^{®} (both Schering); Omniscan^{®}; Feridex^{®}, and Combidex^{®} (all three Advanced Magnetics), Endorem^{®} and Sinerem^{®} (Guerbet), and Clariscan^{®} (Nycomed). A number of various methods of preparation of crystals containing iron (iron oxides) with superparamagnetic properties have been described. These can be classified according to many aspects. Two basic methods of manufacture of superparamagnetic crystals are based on sintering at high temperature and subsequent mechanical disintegration or chemical synthesis in aqueous solution. For applications in medicine, effective particles were produced by wet synthetic techniques; in contrast, sintering is described for production of iron oxides for technological (audio/video media, pigments for dyes, toners) and biotechnological applications such as magnetic separations (Schostek S., Beer A., DE 3,729,697 A1; Borelli N.F., Luderer A.A., Panzarino J.N., U.S. Pat. 4,323,056; Osamu I., Takeshi H., Toshihiro M., Kouji N., JP 60,260,463 A2). The wet chemical synthesis can be divided into a "two-step" synthesis, which first prepares iron oxide-containing nuclei by increasing pH, to which is subsequently added a stabilizer providing physical and other required properties (Kresse M., Pfefferer D., Lawaczeck R., Wagner S., Ebert W., Elste V., Semmler W., Taupitz M. Gaida J., Herrmann A., Ebert M., Swiderski U., U.S. Pat. Appl. 20030185757). In a "one-step" synthesis, iron oxides are prepared by precipitation of iron salts in the presence of a stabilizer, which coats the nuclei in the course of nucleation and thus hinders aggregation and sedimentation of nanocrystals. In addition to classification into "two-step" and "one-step" methods, there exists another differentiation, according to the type of the used solvent, into the methods using water (Hasegawa M., Hokukoku S., U.S. Pat. 4,101,435; Fuji Rebio K.K, JP 59,195,161) or organic solvents (Porath J., Mats L., EP 179,039 A2; Aoyama S., Kishimoto M., Manabe T., Interaction between polymers and magnetic particles - effect on the properties of particulate magnetic recording media, J. Mater. Chem. 2, 277-280, 1992; Norio H., Saturo O., JP 05,026,879 A2). Various order of iron (II) and iron (III) mixing with base influences the amount of produced iron oxide (Kunda W. Rudyk B. DE 2 642 383 A1). The crude product must be always carefully purified and excess admixtures and impurities thus removed. The method of choice is then thermal sterilization. The iron oxides used at present are characterized by particle polydispersity expressed by the polydispersity index, PDI > 1.3. (PDI = *D_{w}*/*D*ₙ, where *D*ₙ = Σ *Di* /*N ₐ D_{w}* =Σ *D*ᵢ⁴/Σ *D*ᵢ³; where N is the number of particles, Di is the diameter of an individual particle). Polydisperse particles have different physical and chemical properties, in contrast to monodisperse ones, the properties of which, including magnetic, are uniform. A drawback of classical magnetite particles also is that they change their properties in air. Their chemical instability causes uncontrolled oxidation with air oxygen, magnetic susceptibility decreases, the colloid loses stability and the nanoparticles aggregate, which is unacceptable for applications in medicine. Therefore, it is better to subject the freshly prepared magnetite particles, immediately after synthesis, to controlled oxidation to maghmite (γ-Fe₂O₃), which is stable in air and does not change its properties.

Generally, the surface of magnetic particles for imaging in medicine is covered by polymers. Almost all nanoparticles commonly used in medicine at present are iron oxides prepared in the presence of polysaccharide dextran as stabilizer (Weissleder R. US 5 492 814 A; Hasegawa M., Hokukoku S., US 4 101 435, Bacic G., Niesman M.R, Bennett H.F., Magin R.L., Schwarz H.M., Modulation of water proton relaxation rates by liposomes containing paramagnetic materials, Magn. Reson. Med. 6, 445-58, 1988; Ohgushi M., Nagayama K., Wada A., Dextran-magnetite: a new relaxation agent and its application to T2 measurements in gel systems, J. Magn. Reson. 29, 599-601, 1978; Pouliquen D., Le Jeune J.J., Perdrisot R., Ermias A., Jallet P., Iron oxide nanoparticles for use as an MRI contrast agent pharmacokinetics and metabolism, Magn. Reson. Imaging 9, 275-283, 1991; Ferrucci J.T., Stark D.D., Iron oxide-enhanced MR imaging of the liver and spleen: review of the first 5 years, Am. J. Roentgenol. 155, 943-950, 1990). Synthesis of such particles is usually performed according to the Molday procedure (Molday R.S. US 4 452 773 A), (Molday R.S., MacKenzie D., Immunospecific ferromagnetic iron-dextran agents for the labelling and magnetic separation of cells, J. Immunol. Methods 52, 353-367, 1982) requiring laborious and costly purification procedures. Dextran, however, is chemically instable, for example it depolymerizes in acid medium and various other reactions may lead to its complete destruction in alkaline medium. Moreover, cells take up the dextran-covered nanoparticles insufficiently, which does not facilitate perfect MR monitoring of cells, probably due to relatively inefficient endocytosis. In addition to dextran, the use of other polysaccharides is described such as arabinogalactan (Josephson L., Groman E.V., Menz E., Lewis J.M., Bengele H., A functionalized superparamagnetic iron oxide colloid as a receptor directed MR contrast agent, Magn. Reson. Imaging 8, 637-646, 1990), starch (Fahlvik A.K., Holtz E., Schroder U., Klaveness J., Magnetic starch microspheres, biodistribution and biotransformation. A new organ-specific contrast agent for magnetic resonance imaging, Invest. Radiol. 25, 793-797, 1990), glycosaminoglycans (Kresse M. EP 0 516 252 A2), (Kresse M., Wagner S., Pfefferer D., Lawaczeck R., Elste V., Semmler W., Targeting of ultrasmall superparamagnetic iron oxide (USPIO) particles to tumor cells in vivo by using transferrin receptor pathways, Magn. Reson. Med. 40, 236-42, 1998) or proteins (Widder D.J., Greif W.L., Widder K.J., Edelman R.R., Brady T.J., Magnetite albumin microspheres: a new MR contrast material, Am. J. Roentgenol. 148, 399-404, 1987) such as albumin, even antibodies (Liberti P.A. ,WO 91/02811) or synthetic polymers such as polymethacrylates and polysilanes. Also transfection agents are described including also poly(amino acid)s (polyalanines, poly(L-arginine)s, DNA of salmon eggs, poly(L-omithine)s), dendrimers, polynucleotides (Frank J.A., Bulte J.W.M., Pat. WO 02100269 A1), polyglutamate, polyimines (Van Zijk P., Goffeney N., Duyn J.H., Bulte J.W.M., Pat. WO 03049604 A3).

Polymer coating considerably increases the particle size, which can affect their penetration and the rate of their metabolic removal in the body. Recently, also dispersions of bare superparamagnetic nanoparticles (polymer-uncoated) for MR imaging were described (Cheng F.-Y., Su C.-H., Yang Y.-S., Yeh C.-S., Tsai C.-Y., Wu C.-L., Wu M.-T., Shieh D.-B., Characterization of aqueous dispersions of Fe3O4 nanoparticles and their biomedical applications, Biomaterials 26, 729-738, 2005). They were prepared in water and stabilized with, e.g., a citrate monomer (Taupitz M., Schnorr J., Wagner S.A., Abramjuk C., Pilgrimm H., Kivelitz D., Schink T., Hansel J., Laub G., Humogen H., Hamm B., Coronary MR angiography: experimental results with a monomer-stabilized blood pool contrast medium, Radiology 222, 120-126, 2002) or tetramethylammonium hydroxide (Cheng F.-Y., Su C.-H., Yang Y.-S., Yeh C.-S., Tsai C.-Y., Wu C.-L., Wu M.-T., Shieh D.-B., Characterization of aqueous dispersions of Fe3O4 nanoparticles and their biomedical applications, Biomaterials 26, 729-738, 2005). The nanoparticles allegedly bring some advantages over those that require a polymer addition to be protected against aggregation.

Stem cells show the ability to differentiate into any specialized cell of the organism and that is why they are in the centre of interest of human medicine, in particular regenerative medicine and cell therapy, where their utilization can be assumed. (Park H.C., Shims Y.S., Ha Y., Yoon S.H., Park S.R., Choi B.H., Park H.S., Treatment of complete spinal cord injury patients by autologous bone marrow cell transplantation and administration of granulocyte-macrophage colony stimulating factor, Tissue Eng. 11, 913-922, 2005; Akiyama Y., Radtke C., Honmou O., Kocsis J.D., Remyelination of the spinal cord following intravenous delivery of bone marrow cells, Glia 39, 229-236, 2002; Akiyama Y., Radtke C., Kocsis J.D., Remyelination of the rat spinal cord by transplantation of identified bone marrow stromal cells, J. Neurosci. 22, 6623-6630, 2002; Hofstetter C.P., Schwarz E.J., Hess D., Widenfalk J., El Manira A., Prockop J.D., Olson, L., Marrow stromal cells form guiding strands in the injured spinal cord and promote recovery, Proc. Natl. Acad. Sci. USA 96, 2199-2204, 2002; Chen J., Li Y., Katakowski M., Chen X., Wang L., Lu D., Intravenous administration of human bone marrow stromal cells induces angiogenesis in the ischemic boundary zone after stroke in rats, Circ. Res 92, 692, 2003; Chen J., Zhang Z.G., Li Y., Wang L., Xu Y.X., Gautam S.C., Intraarterial administration of marrow stromal cells in a rat model of traumatic brain injury, J.Neurosci.Res. 73, 778-786, 2003; Chopp M., Li Y., Treatment of neural injury with marrow stromal cells, Lancet Neurol. 1, 92-100, 2002; Chopp M., Zhang X.H., Li Y., Wang L., Chen J., Lu D., Spinal cord injury in rat: treatment with bone marrow stromal cell transplantation, Neuroreport 11, 3001-3005, 2000; Ramon-Cueto A., Plant G.W., Avila J., Bunge M.B., Long-distance axonal regeneration in the transected adult rat spinal cord is promoted by olfactory ensheathing glia transplants, J. Neurosci. 18, 3803-3815, 1998; Syková E., Urdziková L., Jendelová P., Burian M., Glogarová K., Hájek M., Bone marrow cells - a tool for spinal cord injury repair, Exp. Neurol. 193, 261-262, 2005).

### Disclosure of invention

The subject of the invention is surface-modified superparamagnetic nanoparticle probes based on iron oxides for diagnostic and therapeutical applications. Superparamagnetic nanoparticle probes based on iron oxides, to advantage magnetite or maghemite, with modified surface are formed by a colloid consisting of particles, the size of which ranges from 10 to 30 nm, and their polydispersity index is smaller than 1.3. Their surface is coated with mono-, di- or polysaccharides, amino acids or poly(amino acid)s or synthetic polymers based on (meth)acrylic acid and their derivatives. The saccharides are selected from the group formed by D-arabinose, D-glucose, D-galactose, D-mannose, lactose, maltose, dextrans, dextrins. The amino acid or poly(amino acid) is selected from the group formed by polyalanine, polyglycine, polyglutamine, polyasparagine; polyhistidine, polyarginine, poly(L-lysine), poly(aspartic acid) and poly(glutamic acid). Polymers of derivatives of (meth)acrylic acid are selected from the group containing poly(*N*,*N* dimethylacrylamide), poly(*N*,*N*-dimethylmethacrylamide), poly(*N*,*N*-diethylacrylamide), poly(*N*,*N*-diethylmethacrylamide), poly(*N*-isopropylacrylamide), poly(N-isopropylmethacrylamide). The surface layer of a modification agent amounts to 0.1-30 wt.%, to advantage 10 wt.%, and the iron oxide content to 70-99.9 wt.%, to advantage 90 wt.%. The agents on the surface of particles enable their penetration into cells.

Superparamagnetic nanoparticle probes according to the invention are prepared by preprecipitation of colloidal Fc(OH)₃ by the treatment of aqueous 0.1-0.2M solution of Fe(III) salt, to advantage FeCl₃, with less than an equimolar amount of NH₄OH, at 21 °C, under 2-min sonication at 350 W. To the hydroxide, 0.1-0.2 M solution of a Fe(II) salt, to advantage FeCl₂, is added in the mole ratio Fe(III)/Fe(II) = 2 under 2-min sonicadon and the mixture is poured into five- to tenfold, to advantage eightfold, molar excess of 0.5 M NH₄OH. The mixture is left aging for 0-30 min, to advantage 15 min, and then the precipitate is repeatedly, to advantage 7-10 time, magnetically separated and washed with deionized water of resistivity 18 MΩ·cm⁻¹. In contrast to the present state-of-the-art, 1-3 fold amount, to advantage 1.5 fold amount relative to the amount of magnetite, of 0.1 M aqueous solution of sodium citrate is added and then, dropwise, 1-3 fold amount, to advantage 1.5 fold amount relative to the amount of magnetite, of 0.7 M aqueous solution of sodium hypochlorite. The precipitate is repeatedly, to advantage 7-10 times, washed with deionized water of resistivity 18 MΩ·cm⁻¹, under the formation of colloidal maghemite to which, after dilution, is added
dropwise, possibly under 5-min sonication, an aqueous solution of a modification agent in the weight ratio modification agent/iron oxide 0.1-10, to advantage 0.2 for amino acids and poly(amino acid)s and 5 for saccharides.

The thus prepared nanoparticles reach the size around 10 nm, according to transmission electron microscopy (TEM), with comparatively narrow size distribution characterized by PDI < 1.3. The colloidal stability of the particles in water is a consequence of the presence of the charges originating from Fe(III) and citrate ions.

An essential feature of the preparation of superparamagnetic nanoparticle probes with modified surface according to the invention consists in the fact that slow addition of a solution of modification agent follows precipitation. At that, the modification agent nonspecifically adsorbs on the iron oxide surface. The interaction is a consequence of hydrogen bonds between the polar OH groups of the modification agent and hydroxylated and protonated sites on the oxide surface, or of the agent charge interacting with the citrate complexed on the iron oxide surface. The particles coated with the modification agent do not aggregate as was confirmed by TEM micrographs, according to which the size of surface-modified particles was the same as that of starting iron oxide particles.

Nanoparticles are modified with the agents based on poly(amino acid)s such as polyalanine, polyglycine, polyglutamine, polyasparagine, polyarginine, polyhistidine or polylysine, poly(aspartic acid) and poly(glutamic acid), monosaccharides (e.g. arabinose, glucose, mannose, galactose), disaccharides (e.g. lactose, maltose) and polysaccharides including starch, dextrans and dextrins, and polymers of derivatives of (meth)acrylic acid (e.g. poly(*N*,*N-*dimethylacrylamide), poly(*N*,*N*-dimethylmethacrylamide), poly(*N*,*N*-diethylacrylamide), poly(*N*,*N*-diethylmethacrylamide), poly(*N*-isopropylacrylamide), poly(*N*-isopropylmethacrylamide)).

Superparamagnetic nanoparticle probes with modified surface according to the invention are
designed for labelling of living cells, in particular stem cells. The method will find broad applications in monitoring cells suitable for cell therapy (e.g., stem cells of bone marrow, olfactory glial cells, fat tissue cells). After administration of cells, their fate can be monitored in the recipient body by a noninvasive method, magnetic resonance.

It was found experimentally that the capability of targeting superparamagnetic nanoparticle probes according to the invention in cells is significantly better than with iron oxide particles according to the hitherto used methods. The uptake of poly(amino acid)-modified iron oxide nanoparticles by cells is made possible by their interaction with negatively charged cell surface and subsequent endosomolytic absorption. The nanoparticles are in this way transferred into endosomes, fused with lysosomes under simultaneous destruction of vesicular membrane. Another mechanism of transport of nanoparticle probes into cells may consist in the mannose transporter present on the surface of many types of mammalian cells. Compared with Endorem^{®} (0.11 mg Fe₃O₄, per ml of medium), considerably lower concentrations of iron oxide nanoparticles modified according to the invention were sufficient for complete labelling of cells. An additional advantage is that the patient organism is considerably less loaded with applied particles than it is necessary when using currently commercially available agents.

The invention provides a tool for monitoring the history and fate of cells transplanted into organism including their *in vivo* migration. Nanoparticle probes according to the invention are suitable for determination of diagnoses of pathologies associated with cellular dysfunction. First, the stem cells of the patient are labelled *ex vivo*. In cell labelling, 5-20 µl, to advantage 10 µl, of a colloid containing 0.05-45 mg iron oxide per ml, to advantage 1-5 mg iron oxide per ml of the medium, is added to complete the culture medium and the cells are cultured for 1-7 days, to advantage for 1-3 days, at 37 °C and 5 % of CO₂. During the culturing, the cells fagocytize nanoparticles from the medium to cytoplasm. The thus labelled cells are introduced
into the patient organism, which, when using magnetic field, makes it possible to monitor the movement, localization and survival of exogenous cells by MRI imaging and thus to reveal pathologies associated with cellular dysfunctions.

### Examples

### Example 1

### Preparation of starting (uncoated) superparamagnetic iron oxide nanoparticles

12 ml of aqueous 0.2 M FeCl₃ was mixed with 12 ml of aqueous 0.5 M NH₄OH under sonication (Sonicator W-385; Heat Systems-Ultrasonics, Inc., Farmingdale, NY, USA) at laboratory temperature for 2 min. Then 6 ml of aqueous 0.2 M FeCl₂ was added under sonication and the mixture was poured into 36 ml of aqueous 0.5 M NH₄OH. The resulting magnetite precipitate was left aging for 15 min, magnetically separated and repeatedly (7-10 times) washed with deionized water of resistivity 18 MΩ·cm⁻¹ to remove all residual impurities (including NH₄Cl). Finally, 1.5 ml of aqueous 0.1 M sodium citrate was added under sonication and magnetite was oxidized by slow addition of 1 ml of 5 % aqueous solution of sodium hypochlorite. The above procedure of repeated washing afforded the starting primary colloid.

For determination of the nanoparticle size, dynamic light scattering (DLS) was used, which gave the average hydrodynamic diameter of particles amounting to 90 ± 3 nm, suggesting a narrow size distribution. From TEM micrograph, then *D*ₙ = 6.5 nm a PDI = 1.26. PDI is the polydispersity index characterizing the size distribution width, PDI = *D*_{w}/*D*ₙ, where *D*_{w} and *D*ₙ are the weight- and number-average particle diameter.

### Example 2

### Treatment of superparamagnetic iron oxide nanoparticles with poly(amino acid)s - "two-step synthesis"

To 10 ml of the starting colloid solution containing iron oxide nanoparticles prepared according to Example 1 and diluted to the concentration 2.2 mg iron oxide/ml, 0.01-2 ml (typically 0.2 ml) of aqueous solution of a poly(amino acid) of concentration 0.5-10 mg/ml (typically 1 mg/ml) was added dropwise under stirring and the mixture was sonicated for 5 min.

The poly(amino acid) can be polyalanine, polyglycine, olyglutamine, polyasparagine, polyarginine, polyhistidine or poly(L-lysine), poly(aspartic acid) and poly(glutamic acid).

### Example 3

### Treatment of superparamagnetic iron oxide nanoparticles with saccharides - "two-step synthesis"

Various volumes (0.1-5 ml) of 4 wt.% aqueous solution of a saccharide were added dropwise under stirring to 10 ml of the starting colloid solution containing iron oxide nanoparticles prepared according to Example 1, diluted to the concentration 2.2 mg iron oxide/ml and the mixture was sonicated for 5 min. The particles were repeatedly washed.

The saccharide can be D-arabinose, D-glucose, D-galactose, D-mannose, lactose, maltose, dextrans, dextrins.

### Example 4

### Treatment of superparamagnetic iron oxide nanoparticles with (meth)acrylic acid derivatives - "two-step synthesis"

To an 0.003-0.07 wt.% (typically 0.03 wt.%) solution of 4,4'-azobis(4-cyanopentanoic acid) was added a corresponding amount of the colloid containing 0.1-2 g (typically 0.5 g) of particles prepared according to Example 1 so that the total volume of the mixture was 30 ml. To the solution was added 0.1-2 (typically 1) g of a (meth)acrylic acid derivative, the solution was bubbled with nitrogen for 10 min and heated at 70 °C for 8 h under stirring (400 rpm). The resulting product was repeatedly (3-5 times) magnetically separated or centrifuged (14 000 rpm), washed with water or isotonic 0.15 M sodium chloride and sonicated until the formation of a colloidal solution.

The (meth)acrylic acid derivative can be poly(*N*,*N*-dimethylacrylamide), poly(*N*,*N-*dimethylmethacrylamide), poly(*N*,*N*-diethylacrylamide), poly(*N*,*N*-diethylmethacrylamide), poly(*N*-isopropylacrylamide), poly(*N*-isopropylmethacrylamide).

### Example 5

### Example 5

### Optical microscopy of labelled cells

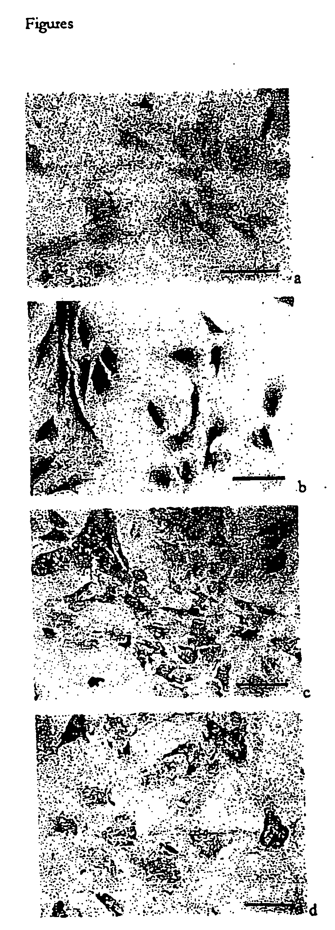

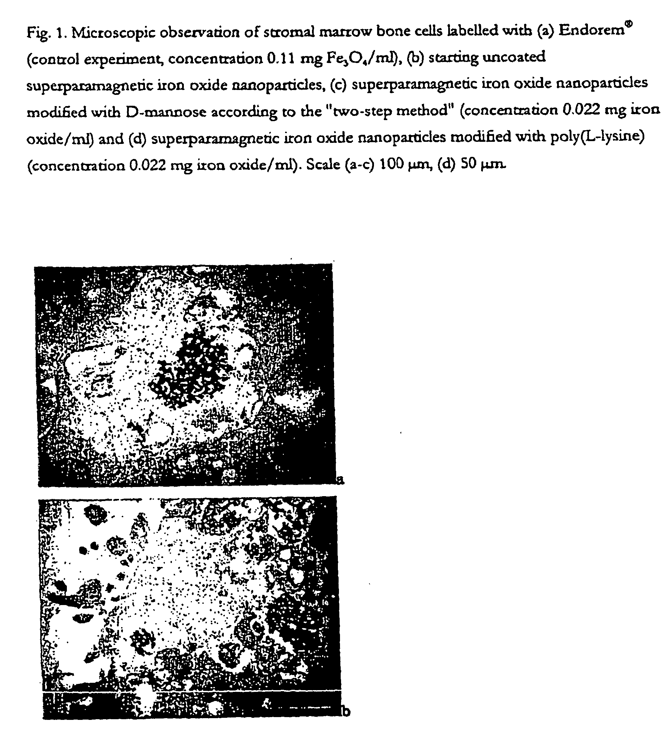

Stromal cells of bone marrow (MSC) of rat labelled by both starting uncoated and surface-modified superparamagnetic iron oxide nanoparticles were observed in optical microscope. The cells labelled with Endorem^{®} (0.11 mg Fe₃O_{4/}ml) served as control (Fig. 1a). A drawback of Endorem^{®} was its tendency to adhere to the cell surface; moreover, it sticked also to the bottom of vessel.

The cells in contact with starting (uncoated) nanoparticles prepared according to Example 1 proliferated and approximately one of every ten cells endocytized iron oxide nanoparticles of iron oxide (Fig. 1 b).

From observation of the cells in contact with superparamagnetic nanoparticles modified with D-mannose by the "two-step method" (after the synthesis) according to Example 3, the
optimum concentration of D-mannose added to the colloid was assessed amounting to 12.8 mg D-mannose per ml of the colloid, which ensures labelling of ca. 50 % of cell population (Fig. 1c)

Maximum labelling of cells (almost 100 %) was achieved with poly(L-lysine)-modified nanoparticles (0.02 mg poly(L-lysine) per ml colloid (Fig. 1d)).

### Example 6

### Transmission electron microscopy of cells labelled with superparamagnetic iron oxide nanoparticles

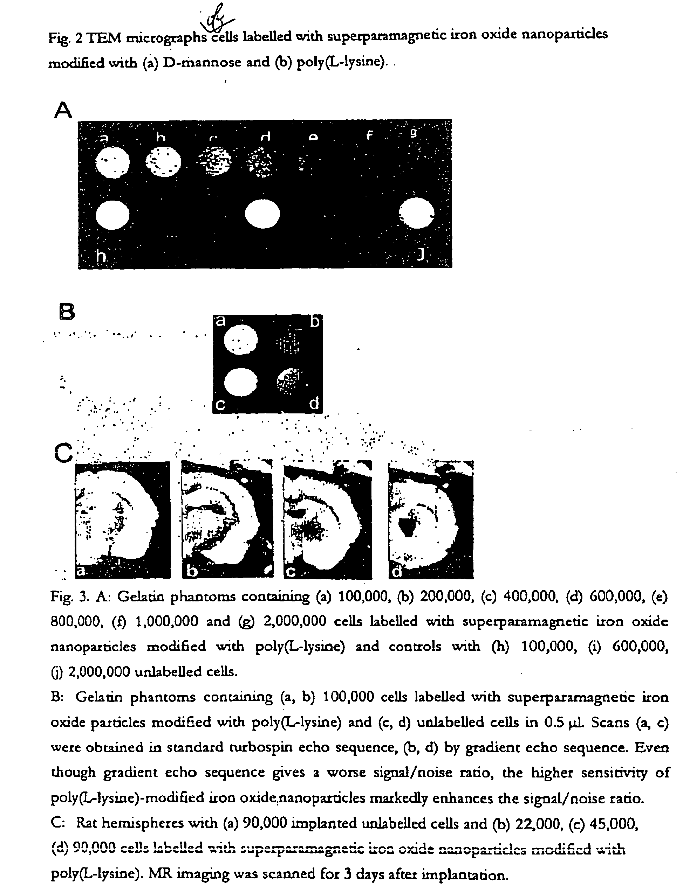

Transmission electron micrograph of MSC cells labelled with superparamagnetic nanoparticles of iron oxide modified with D-mannose according to Example 3 and with poly(L-lysine) (PLL) according to Example 2 is shown in Fig. 3. Numerous aggregates of both types of superparamagnetic nanoparticles inside cells labelled with nanoparticles modified with both D-mannose and poly(L-lysine) are visible. The nanoparticle aggregates were evenly distributed in cell cytoplasm; their accumulation on cell membranes was not perceptible.

### Example 7

### Quantitative determination of cells labelled with superparamagnetic iron oxide nanoparticles

Superparamagnetic iron oxide nanoparticles modified with both poly(L-lysine) according to Example 2 and with D-mannose according to Example 3 were successfully endocytized by MSC cells (as follows from Figs. 1 and 2). MSC cells were cultivated in duplicate on uncoated six-well culture plates at the density 10⁵ cells per mm². Endorem^{®} and the nanoparticles modified with poly(L-lysine) or D-mannose were added to culture medium (10 µl/ml) and the cells incubated for 72 h. After washing out excess contrast substance with the culture medium, the cells were fixed with 4 % solution of paraformaldehyde in 0.1 M phosphate buffer (PBS) and tested for iron under the formation of iron(III) ferrocyanide (Prussian Blue). The number of labelled and unlabelled cells was determined in an inverted light microscope (Axiovert 200, Zeiss) by counting randomly selected five fields per well and two wells per each run (Table 1). The cells in each image were manually labelled as Prussian Blue-positive or negative; the number of labelled cells was then counted using the image analysis toolbox in
program Matlab 6.1 (The MathWorks, Natick, MA, USA). The best labelling of cells was obtained with nanoparticles containing 0.02 mg poly(L-lysine) per ml of colloid.

**Table 1. Percentage of stromal cells of bone marrow (MSC) labelled in vitro with superparamagnetic nanoparticles**

| | Uncoated iron oxide | PLL-modifid iron oxide (0.02 mg PLL/ml) | D-Mannose-modified iron oxide | Endorem^{®} |
|---|---|---|---|---|
| MSC (rat) | 27.9 | 92.2 | 50.8 | 60.0 |
| MSC (human) | not tested | 87.5 | not tested | 65.2 |

### Example 8

### Relaxivity of cells labelled with superparamagnetic iron oxide nanoparticles modified with poly(L-lysine)

To further verify the presence of poly(L-lysine)-modified superparamagnetic iron oxide nanoparticles prepared according to Example 2 in bone marrow cells (MSCs), samples with suspension of Endorem^{®} and poly(L-lysine)-modified superparamagnetic nanoparticles in a 4% gelatin solution and samples with suspensions of Endorem^{®}-labelled cells and poly(L-lysine)-modified superparamagnetic nanoparticles with various amounts of cells in gelatin solution were prepared. Subsequently, relaxation times of samples were measured and their MR images were obtained.

For determination of relaxation times *T*₁ a *T*₂, a relaxometer Bruker Minispec 0.5 T was used. The values were recalculated to proton relaxivities R₁=1/*T*₁, R₂ = 1/*T*₁ and related to real concentrations *r*₁= R₁/c (s⁻¹/mmol), *r*₂ = R₂/c (s⁻¹/mmol), or they were related to the number of cells in 1 ml, where R₂ and R₁ are corrected for gelatin. The relaxivity values are given in Tables 2 and 3. From Table 3 follows that the r₂ value of poly(L-lysine)-modified superparamagnetic iron oxide nanoparticles according to Example 2 is considerably higher than with Endorem^{®}.

**Table 2. η values of poly(L-lysine)-modiried superparamagnetic iron oxide nanoparticles (PLL) and Endorem^{®}**

| | Relaxivity *r*₁ of suspension of contrast agent in gelatin | Relaxivity *r*₁ of suspension of labelled cell in gelatin |
|---|---|---|
| | (s⁻¹/mmol Fe) | (s⁻¹/10⁶ cells per ml) |
| PLL-modified | | |
| iron oxide | 17.4 | 0.32 |
| Endorem | 19.6 | 0.18 |

**Table 3. r₂ values of poly(L-lysine)-modified superparamagnetic iron oxide nanoparticles (PLL) and Endorem^{®}**

| | Relaxivity *r*₂ of contrast material suspension in gelatin | Relaxivity r₂ of labelled cell suspension in gelatin |
|---|---|---|
| | (l⁻¹/mmol Fe) | (s⁻¹/10⁶ cells per ml) |
| PLL-modified | | |
| iron oxide | 213 | 4.29 |
| Endorem^{®} | 126 | 1.24 |

The average iron content determined spectrophotometrically after mineralization amounted to 35.9 pg Fe per cell in poly(L-lysine)-modified superparamagnetic iron oxide nanoparticles and 14.6 pg Fe per cell in Endorem^{®}-labelled cells

### Example 9

***In vitro* MR imaging of cells labelled with superparamagnetic nanoparticle probes** Imaging of labelled cells *in vitro* is advantageous for proof of MRI sensitivity and, at the same time, for imitating the course of the signal in brain tissue. Rat MSC cells were labelled with poly(L-lysine)-modified superparamagnetic iron oxide nanoparticles according to Example 2 and a cell suspension in a 4 % gelatin solution of concentration 4,000, 2,000, 1,600, 1,200, 800, 400 and 200 cells per µl was prepared. The unlabelled MSC rat cells were suspended in a 4 % gelatin solution of concentration 4,000, 1,200 and 200 cells per µl.

The cell samples were subsequently imaged with a 4.7 T Bruker spectrometer using standard turbospin sequence (sequence parameters: repetition time TR = 2 000 ms, effective echo-time TE = 42.5 ms, turbo factor = 4, number of acquisitions AC = 16, image field FOV = 64 x 64 mm, matrix MTX = 512 x 512, layer thickness 0.75 mm; the set geometry affords a comparable size of voxel as in *in vivo* measurement) and the gradient echo sequence (TR = 180 ms, TE = 12 ms, the same geometry of imaging).

When using both sequences, the cells labelled with poly(L-lysine)- or D-mannose-modified superparamagnetic iron oxide nanoparticles modified with poly(L-lysine) (Fig. 3 A, B) or D-mannose afford an excellent contrast compared with unlabelled cells. A visible contrast in MR image was observed also in a sample, each image voxel of which contained mere 2.3 cells on average. A similar series of experiments were given in the preceding work (Jendelová. P., Herynek V., DeCroos J., Glogarová K, Andersson B., Hájek M., Syková E., Imaging the fate of implanted bone marrow stromal cells labeled with superparamagnetic nanoparticles, Magn. Reson. Med. 50, 767-776, 2003), where MR imaging of gelatin phantoms showed a hypointensive signal at concentrations above 625 cells per µl.

### Example 10

### In vivo MR imaging of cells labelled with superparamagnetic nanoparticle probes

Wistar rats were in the course of measurement. The rats were monitored for 3 days after transplantation in a Broker 4.7 T spectrometer equipped with a surface coil of domestic production. Simple sagital, coronal and transverse scans were obtained by a fast gradient echo sequence for localization of subsequent *T*₂- and *T*₂*-weighted images measured by standard turbospin sequence (TR = 2 000 ms, TE = 42.5 ms, turbo factor = 4, AC = 16, FOV = 30 x 30 mm, matrix MTX 256 x 256, layer thickness 0.75 mm) and gradient echo sequence (TR = 180 ms, TE = 12 ms, the same geometry of imaging). Figure 3C proves that cells labelled with poly(L-lysine)-modified superparamagnetic iron oxide nanoparticles according to Example 2 were clearly discernible also *in vivo.* Unlabelled cell implants were visible in MR images as a tissue inhomogeneity without a hypotensive signal (Fig. 3C).

### Industrial applicability

The invention can be exploited in human and veterinary medicine, biology and microbiology.

## Claims

1. Method of preparation of superparamagnetic nanoparticle probes **characterized in that** colloidal Fe(OH)₃ is preprecipitated by the treatment of aqueous 0.1-0.2 M solution of Fe(III) salt, to advantage FeCl₃, under sonication, with less than an equimolar amount of NH₄OH, at 21 °C, to which 0.1-0.2 M solution of a Fe(II) salt, to advantage FeCl₂, is added in the mole ratio Fe(III)/Fe(II) = 2 and the mixture is poured into five- to tenfold, to advantage eightfold, molar excess of 0.5 M NH₄OH, the mixture is left aging for 0-30 min, to advantage for 15 min, then the precipitate is repeatedly, to advantage 7-10 times, magnetically separated and washed with deionized water of resistivity 18 MΩ cm⁻¹, then a 1-3 fold amount, to advantage 1.5 fold amount relative to the amount of magnetite, of 0.1 M aqueous solution of sodium citrate is added under sonication and then, dropwise, 1-3 fold amount, to advantage 1.5 fold amount relative to the amount of magnetite, of 0.7 M aqueous solution of sodium hypochlorite. The precipitate is repeatedly, to advantage 7-10 times, washed with deionized water of resistivity 18 MΩ cm⁻¹, under the formation of colloidal maghemite to which, after dilution, is added dropwise, possibly under 5-min sonication, an aqueous solution of a modification agent in the weight ratio modification agent/iron oxide 0.1-10, to advantage 0.2 for poly(amino acid)s and 5 for saccharides.

2. Superparamagnetic nanoparticle probes based on iron oxides, to advantage magnetite or maghemite, with modified surface obtained with the method according to claim 1, **characterized in that** they are coated with mono-, di- or polysaccharides from the group including D-arabinose, D-glucose, D-galactose, D-mannose, lactose, maltose or poly(amino acid)s from the group including polyalanine, polyglycine, polyglutamine, polyasparagine, polyhistidine, polyarginine, poly(L-lysine), poly(aspartic acid) and poly(glutamic acid) or polymers of (meth)acrylic acid derivatives from the group containing poly(*N,N*-dimethylacrylamide), poly(*N,N*-dimethylmethacrylamide), poly(*N,N*-diethylacrylamide), poly(*N,N*-diethylmethacrylamide), poly(*N*-isopropylacrylamide), poly(*N*-isopropylmethacrylamide) and form a colloid consisting of particles with narrow size distribution with polydispersity index lower than 1.3, the average size of which ranges from 10 to 30 nm, iron oxide content amounts to 70-99.9 wt.%, to advantage 90 wt.%, the modification agent content makes 0.1-30 wt.%, to advantage 10 wt.%.

## Patentansprüche

1. Verfahren zur Herstellung superparamagnetischer Nanopartikelsonden, **dadurch gekennzeichnet, dass** kolloidales Fe(OH)₃ durch die Behandlung von 0,1 - 0,2 M wässeriger Lösung eines Fe(III)-Salzes, vorzugsweise von FeCl₃, unter Ultraschallbehandlung mit weniger als einer äquimolaren Menge an NH₄OH bei einer Temperatur von 21 °C ausgefällt wird, zu der eine 0,1 -0,2 M Lösung eines Fe(II)-Salzes, vorzugsweise FaCl₂, in einem Molverhältnis Fe(III)/Fe(II) = 2 hinzugefügt wird, das Gemisch in einen 5- bis 10-fachen, vorzugsweise 8-fachen, molaren Überschuss von 0,5 M NH₄OH gegeben wird, das Gemisch für 0 - 30 min, vorzugsweise 15 min, ruhen gelassen wird, worauf der Niederschlag mehrfach, vorzugsweise 7- bis 10-mal magnetisch getrennt und mit entionisiertem Wasser mit einem Widerstand von 18 MΩcm⁻¹ gewaschen wird, dann eine 1- bis 3-fache Menge, vorzugsweise eine 1,5-fache Menge, bezogen auf die Magnetitmenge, einer 0,1 M wässerigen Lösung von Natriumcitrat unter Ultraschallbehandlung hinzugefügt wird und dann tropfenweise eine 1- bis 3-fache Menge, vorzugsweise eine 1,5-fache Menge, bezogen auf die Magnetitmenge, einer 0,7 M wässerigen Lösung von Natriumhypochlorit hinzugefügt wird, und der Niederschlag wiederholt, vorzugsweise 7- bis 10-mal, mit entionisiertem Wasser mit einem Widerstand von 18 MΩcm⁻¹ unter Bildung von kolloidalem Maghemit gewaschen wird, zu dem nach Verdünnung tropfenweise, gegebenenfalls unter einer 5-minütigen Ultraschallbehandlung, eine wässerige Lösung eines Modifikators in einem Gewichtsverhältnis Modifikator/Eisenoxid von 0,1-10, vorzugsweise 0,2 für Polyaminosäuren und vorzugsweise 5 für Saccharide, hinzugefügt wird.

2. Superparamagnetische Nanopartikelsonden auf der Basis von Eisenoxiden, vorzugsweise Magnetit oder Maghemit, mit einer durch das Verfahren gemäß Anspruch 1 modifizierten Oberfläche, **dadurch gekennzeichnet, dass** die Nanopartikel mit Mono-, Di- oder Polysacchariden aus der Gruppe umfassend D-Arabinose, D-Glucose, D-Galactose, D-Mannose, Lactose und Maltose, oder Polyaminosäuren aus der Gruppe umfassend Polyalanin, Polyglycin, Polyglutamin, Polyasparagin, Polyhistidin, Polyarginin, Poly-L-lysin), Polyasparaginsäure und Polyglutaminsäure, oder Polymeren von (Meth)acrylsäurederivaten aus der Gruppe Poly(N,N-dimethylacrylamid), Poly(N,N-dimethylmethacrylamid), Poly(N,N-diethylacrylamid), Poly(N,N-diethylmethacrylamid), Poly(N-isopropylacrylamid), Poly(N-isopropylmethacrylamid) beschichtet sind, und die Nanopartikel ein Kolloid bilden, bestehend aus Partikeln mit einer engen Größenverteilung mit einem Polydispersitätsindex kleiner als 1,3, wobei die mittlere Größe im Bereich von 10 bis 30 nm liegt, und der Eisenoxidgehalt 70 bis 99,9 Gew.-%, vorzugsweise 90 Gew.-%, beträgt, und der Modifikatorgehalt 0,1 -30 Gew.-%, vorzugsweise 10 Gew.-%, ausmacht.

## Revendications

1. Méthode de préparation de sondes à nanoparticules super paramagnétiques **caractérisée en ce que** du Fe(OH)₃ colloïdal est préprécipité par le traitement d'une solution aqueuse de 0.1 à 0.2M de sel de Fe(III), de préférence de FeCl₃, sous ultrasons, avec une quantité inférieure à une quantité équimolaire de NH₄OH, à 21°C, puis on ajoute une solution de 0.1 à 0.2 M de sel de Fe(II), de préférence de FeCl₂, dans un rapport molaire de Fe(III)/Fe(II) égal à 2, et le mélange est versé dans un excédent molaire d'un facteur 5 à 10, de préférence d'un facteur 8, de NH₄OH 0.5 M, on laisse agir le mélange pendant 0 à 30 min, de préférence pendant 15 min, et ensuite, à plusieurs reprises, de préférence 7 à 10 fois, le précipité est séparé magnétiquement et est lavé avec de l'eau déminéralisée ayant une résistivité de 18 MΩ cm⁻¹. Ensuite une quantité d'une solution aqueuse 0.1M de citrate de sodium de 1 à 3 fois, de préférence de 1.5 fois, la quantité de magnétite est ajoutée en appliquant des ultrasons et ensuite, goutte à goutte, une quantité d'une solution aqueuse 0.7 M d'hypochlorite de sodium de 1 à 3 fois, de préférence 1.5 fois, la quantité de magnétite. Le précipité est lavé à plusieurs reprises, de préférence 7 à 10 fois, avec de l'eau déminéralisée ayant une résistivité de 18 MΩ cm⁻¹, jusqu'à la formation de maghémite colloïdale à laquelle, après dilution, on ajoute goutte à goutte, éventuellement en appliquant des ultrasons pendant 5 min, une solution aqueuse d'un agent de modification, dans un rapport en poids de l'agent de modification/oxyde de fer compris entre 0.1 et 10, de préférence égal à 0.2 pour les poly(acides aminés) et à 5 pour les saccharides.

2. Sondes à nanoparticules super paramagnétiques à base d'oxydes de fer, de préférence de magnétite ou de maghémite, ayant une surface modifiée obtenue à l'aide de la méthode selon la revendication 1, **caractérisées en ce qu'**elles sont recouvertes de monosaccharides, de disaccharides ou de polysaccharides provenant du groupe comprenant le D-arabinose, le D-glucose, le D-galactose, le D-mannose, le lactose et le maltose, ou recouvertes de poly(acides aminés) provenant du groupe comprenant la polyalanine, la polyglycine, la polyglutamine, la polyasparagine, la polyhistidine, la polyarginine, la poly(L-lysine), le poly(acide aspartique) et le poly(acide glutamique) ou recouvertes de polymères de dérivés d'acide (méth)acrylique provenant du groupe contenant le poly(*N,N*-diméthylacrylamide), le poly(*N,N-*diméthylméthacrylamide), le poly(*N,N*-diéthylacrylamide), le poly(*N,N*-diéthylméthacrylamide), le poly(*N*-isopropylacrylamide), le poly(*N*-isopropylméthacrylamide), et forment un colloïde constitué de particules ayant une répartition granulométrique étroite avec un indice de polydispersité inférieur à 1.3, dont la taille moyenne est comprise entre 10 et 30 nm, dont la teneur en oxyde de fer est de 70 à 99,9 % en poids, de préférence de 90 % en poids, et dont la teneur en agent de modification est de 0.1 à 30 % en poids, de préférence de 10 % en poids.
